# EUROPEAN PATENT APPLICATION

(11) **EP 2 990 069 A1**
(43) Date of publication of application: **02.03.2016**
(21) Application number: 15275193.9
(22) Date of filing: 28.08.2015
(51) Int. Cl.: A61M 25/10

(54) **SHAPED OR TEXTURED MEDICAL BALLOON**

(30) Priority: 01.09.2014 GB 201415424; 07.11.2014 GB 201419873
(71) Applicant: Cook Medical Technologies LLC, Bloomington, IN 47404 (US)
(72) Inventor: Aggerholm, Steen, 4660 St. Heddinge (DK); Lysgaard, Thomas, 2680 Solroed Strand (DK)
(74) Representative: Williams Powell

(57) **Abstract**

A medical balloon (30) includes a balloon wall (60) in a plurality of strips (50) of amorphous polymer material. The strips (50) may be textured, for example roughened, or shaped. The texturing or shaping of the strips (50) is retained upon inflation of the balloon (30) to inflation pressures, producing a structure which will ensure reliable positioning of the balloon (30) in a vessel and reliable retention of a medical device on the balloon. Moreover, the strips (50) can increase the tear resistance of the balloon (30) in particular can prevent the propagation of circumferential tears around the balloon (30).

## Description

### Technical Field

The present invention relates to a medical balloon and in some embodiments to a strengthened medical balloon having shaped and/or textured properties.

### Background Art

Medical balloons are well known in the medical field and are used, for example, in the deployment of implantable medical devices such as stents and stent grafts, for angioplasty procedures, for temporary vessel occlusion, in valve repair procedures and so on.

It is generally optimal to make medical balloons with as thin a balloon wall as possible, which reduces the deflated footprint of the balloon for delivery purposes, as well as optimising the flexibility of the deflated balloon.

A standard single layer or multi layered balloon tends to have a very smooth outer surface when inflated, as a result of the inflation pressure acting to smoothen any surface texturing of the balloon. As a result, such balloons tend to be slippery, which means that they are able to move from their deployed position in a vessel or across a valve. When used to carry a medical device such as a stent or stent graft, the medical device may slip on the balloon during the deployment procedure, causing this to fail to deploy properly, for example to be deployed in an incorrect position in a vessel or not to open fully.

Various attempts have been made to try to give a balloon a roughened or textured outer surface. However, these attempts have not been universally successful. For example, as indicated above, any texturing of a balloon tends to be smoothed out when the surface of the balloon is stretched during inflation. Attaching texturing elements or a texturing layer to a balloon can avoid the problems of smoothing by stretch but generally make the balloon more complex, increasing the volume of the balloon material and as a result its deflated footprint, as well as reducing the flexibility of the deflated balloon.

Furthermore, it is often necessary to operate medical balloons at elevated pressures, for example to open a vessel or remove plaque or other build up. So doing, however, increases the risk of the balloon bursting. While some balloon burst is manageable, any circumferential burst can result in fragmentation of the balloon material and loss of fragments into the bloodstream, which should be avoided at all costs. While a strengthening sleeve, for example, can avoid fragmentation and even burst of a balloon, this adds layers to the balloon and as a result volume of balloon material, with the disadvantages noted above.

Balloons are also difficult to image during deployment, as a result of the materials used in the balloon. Attempts have been made to provide some form of radiopacity to the balloon, for example by inflating the balloon with contrast media, by embedding radiopaque elements into the balloon and so on. Contrast media can be toxic and therefore not ideal in situations where there is risk of balloon burst, while embedding of radiopaque elements can weaken the balloon wall and increase the risk of balloon rupture or otherwise result in increased volume of balloon material.

Some examples of medical balloons are disclosed in US-2013/0261548, US-2013/0053770, US-8,048,028, US-2005/0004649, US-2007/0100279, US-5,081,997 and US-2007/010363.

### Disclosure of the Invention

The present invention seeks to provide an improved medical balloon and in the preferred embodiments a medical balloon having texturing and/or a non-cylindrical shape.

According to an aspect of the present invention, there is provided a medical balloon, including: a balloon body member, the balloon body member having first and second ends, a longitudinal dimension and a body length, and a balloon wall formed of polymer material; at least one strip extending between the first and second ends of the body member, the at least one strip comprising amorphous polymer material; the at least one strip being at least one of textured and shaped.

The at least one strip is preferably fixed to and disposed in overlying relationship on the balloon wall.

The at least one strip, it has been found, will retain its texturing or shape even on inflation of the balloon to a high pressure, which does not occur with prior art structures. In embodiments, the remainder of the balloon surface, specifically the exposed parts of the balloon body portion, will generally inflate to be smooth. Textured strips, which could for example be roughened, are able to assist in holding the balloon in position within a vessel or across a valve, for example, as well as to hold a medical device, such as a stent or stent graft, correctly in place on the balloon.

It has been found that amorphous polymer material enables the at least one strip to retain its texturing or shape reliably, even on inflation of the balloon. The amorphous polymer material can also be rigid but easy to shape and/or texture as desired during manufacture of the balloon.

Furthermore, as will be apparent from the teachings herein, the strips of the preferred embodiments can be constructed to act as strengthening elements able to stop circumferential propagation of balloon tears. As a result, any tear in the balloon wall will tend to propagate longitudinally along the length of the balloon with significantly reduced risk of loss of balloon fragments. This allows the use of a thin balloon in order to optimise balloon flexibility and footprint for deployment.

In the preferred embodiment, the medical balloon includes a plurality of strips, preferably generally evenly spaced circumferentially around the balloon.

Advantageously, the or each strip extends parallel to the longitudinal direction of the balloon body member. The advantage of this feature, especially in embodiments in which the strips are harder or more rigid than the balloon wall, is that the strips do not adversely affect the foldability and wrappability of the balloon for deployment purposes.

In a typical embodiment, the balloon includes tapered end portions and balloon necks, the or each strip preferably extending along at least the tapered end portions. In practice, the or each strip may extend also to the necks of the balloon. The advantage of having the strip or strips extend into the end cones of the balloon, and preferably also to the necks, is that the strips can in the preferred embodiments act as strengthening elements along a greater extent of the balloon and in particular along those portions of the balloon where the balloon wall is thinnest.

In some embodiments, the amorphous polymer material has embedded therein a heat conductive particulate material.

In an embodiment, the or each strip is formed of a polymer material having between 25 to 90% by weight of heat conductive particulate material, preferably between 60 to 85% and in a preferred embodiment around 80% by weight of heat conductive particulate material. A concentration of around 80% can be used, although in some embodiments a lower concentration is preferred, for instance of around 25%.

Advantageously, the or each strip has a flattened outer surface, in particular may have a height, that is above the outer surface of the balloon wall, of around 0.05 to 0.2 mm. In other words, the strips do not significantly affect the outer perimeter and shape of the balloon.

In a preferred embodiment, the or each strip is formed of an amorphous polymer material.

The amorphous polymer material is preferably an amorphous polyamide material, preferably an amorphous polyamide 12-based copolymer, preferably an amorphous Nylon material such as amorphous Nylon 12. One example is Grilamid TR55. Amorphous polymer materials, especially amorphous polyamide materials and Nylon materials, can provide very rigid elements which can easily be textured and/or shaped during manufacture of a balloon.

The balloon body member, in particular the balloon wall, or at least a layer thereof, can comprise or be formed of a polymer material, preferably a non-amorphous polymer material, which can be of the same polymer type and even can include the same polymer as the at least one strip. For example, in a preferred embodiment, the or each strip can comprise or be formed of an amorphous Nylon 12 material and the balloon wall can comprise or be formed of a non-amorphous Nylon 12 material.

In one embodiment, the or each strip comprises or is formed of an amorphous polymer material having a greater hardness than the hardness of the polymer material forming the balloon body member.

In one embodiment, the or each strip comprises or is formed of Nylon 6.6 or Nylon 6 whereas the balloon body member comprises or is formed of Nylon 12.

Preferably, the balloon wall and the at least one strip are co-extruded.

In some embodiments, the strips can have a hardening additive, or the balloon wall can have a softening additive. This feature may enhance the strengthening characteristics of the strips. In some embodiments at least, the presence of a powder of heat conductive particulate material may contribute to the hardness or rigidity of the strips and/or may make them more easily smashed, shaped or textured.

It is not excluded, in all embodiments, however, that the strips could be made of a softer material, for example with a polymer which is softer than the polymer used for the balloon wall.

Tungsten has been found to be a suitable heat conductive particulate material as it may assist with the desired reliable texturing and/or shaping and is also radiopaque.

In an embodiment, the or each strip has a concave shape between the first and second ends of the balloon. Such a shape is useful in holding the balloon across a valve and also in holding a medical device in position on the balloon.

In some preferred embodiments, the or each strip has a greater tear resistance than a tear resistance of the balloon wall.

In some embodiments, each of the at least one strip has a base which extends to the outer wall surface but not into the balloon wall. This can enable the balloon wall to be strong, for example by being made of a non-amorphous polymer material. However, in other embodiments, each of the at least one strip has a base which extends into the balloon wall, for example part way or all the way through the balloon wall.

In some embodiments, each of the at least one strip has an inflated strip thickness in an inflated condition of the balloon and the balloon wall has an inflated wall thickness in an inflated condition of the balloon, and the inflated strip thickness of each of the at least one strip is greater than the inflated wall thickness.

The skilled person will appreciate that the terms 'inflated wall thickness' and 'inflated strip thickness' refer to thicknesses in an inflated condition of the balloon and do not necessarily mean that the thicknesses themselves are inflated.

In preferred embodiments, the inflated strip thickness of each of the at least one strip is at least twice, at least four times or most preferably at least six times the inflated wall thickness. Having this greater thickness is advantageously able to stop circumferential tears from propagating. These ratios can be with respect to the thinnest point of the inflated wall thickness. However, in other embodiments they can be with respect to the thickest point of the inflated wall thickness or an average inflated wall thickness.

According to another aspect of the present invention, there is provided a method of forming a medical balloon including the steps of:
forming a raw balloon tubing by: a) forming a tube of balloon material; b) applying to the tube of balloon material at least one strip of or comprising amorphous polymer material;
placing the raw balloon tubing in a mold having an inner surface in the shape of a medical balloon;
heating and inflating the raw tubing in the mold, thereby to cause the raw tubing to inflate to the inner surface of the mold, wherein the at least one strip is flattened between the mold surface and the balloon material, as well as being at least one of textured and shaped; and
removing the formed balloon from the mold.

Preferably, the tube and at least one strip are coextruded for example to provide a unitary balloon.

As discussed above, it has been found that amorphous polymer material can be rigid and easily shaped. It also retains its shape or texture even on inflation of the balloon.

Preferably, the inner mold surface is roughened or textured, whereby inflation of the raw tubing causes the outer surface of the balloon and of the or each strip to be roughened or textured.

In an embodiment, the inner mold surface has a varying diameter along a length thereof, wherein the raw tubing inflates to a varying diameter so as to cause the or at least one of the strips to have a non-straight inflated shape.

### Brief Description of the Drawings

Embodiments of the present invention are described below, by way of example only, with reference to the accompanying drawings, in which:
Figure 1 is a schematic diagram showing the principal parts of an example of balloon catheter;
Figure 2 is a schematic diagram of an embodiment of medical balloon according to the present invention;
Figure 3 is a cross-sectional view of the medical balloon of Figure 2 in a fully inflated condition;
Figure 4 is a schematic diagram of a cross-sectional view of the medical balloon of Figure 3 as formed in a mold;
Figures 5 and 6 are schematic diagrams of a medical balloon according to the diagrams of Figures 2 and 3 when inflated to a relatively low pressure;
Figure 7 is a schematic diagram of another embodiment of medical balloon similar to the embodiment of Figures 2 and 3, showing the balloon in a fully inflated condition;
Figure 8 is a schematic diagram of another embodiment of, shaped, medical balloon according to the present invention;
Figure 9 is a schematic diagram of an example of mold assembly for forming a medical balloon according to the teachings herein;
Figure 10 is a cross-sectional view of an embodiment of raw tubing for forming a medical balloon according to the present invention;
Figure 10A is a cross-sectional view of an embodiment of raw tubing for forming a medical balloon according to the present invention;
Figure 11 is a cross-sectional view of another embodiment of raw tubing for forming a medical balloon according to the present invention;
Figures 12A to 12F show other embodiments of raw tubing for forming a medical balloon according to the teachings herein; and
Figure 13 is a transverse cross-sectional view of an embodiment of a balloon in a delivery device.

### Description of the Preferred Embodiments

The accompanying drawings are schematic only. It is to be understood that the dimensions and proportions of the various components of the devices shown in the drawings are not to scale or in proportion relative to one another. It is also to be understood that the drawings depict only the principal components of the device shown therein and that other elements and components of the device which are not central to understanding the teachings herein have been omitted for the sake of clarity.

The teachings herein are applicable to any medical balloon including, for example, vessel occlusion balloons, delivery balloons used for delivering implantable medical devices, angioplasty balloons, valve treatment balloons and so on.

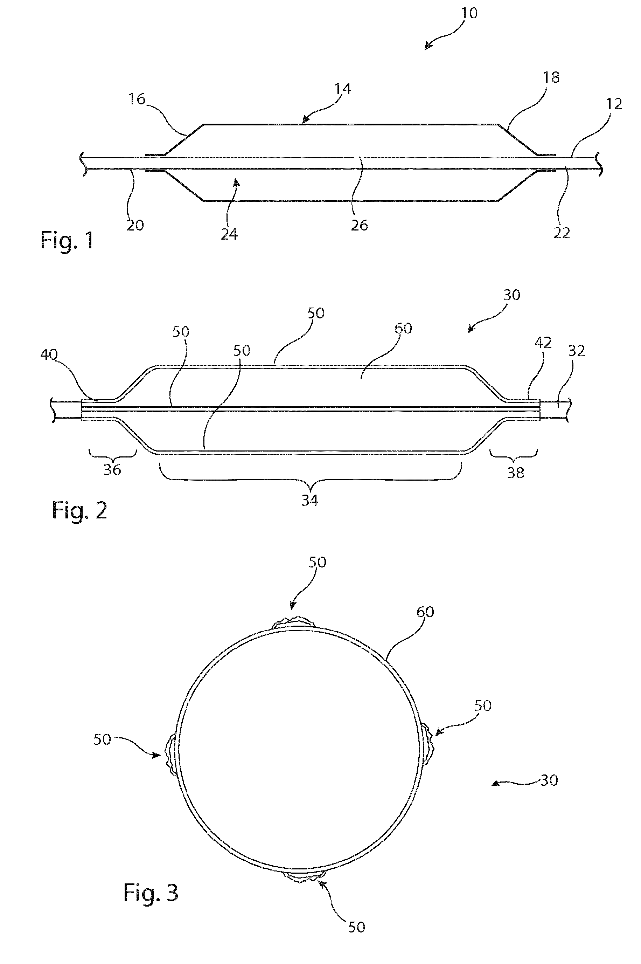

Referring first to Figure 1, this shows an embodiment of medical balloon 10 which is fitted to a balloon catheter 12, which may be of any known form. The balloon 10 includes a body portion 14 which in this embodiment is substantially cylindrical along its length and is annular in transverse cross-section. At either end of the body portion 14, the balloon includes end cones 16, 18 which taper towards necks 20 and 22 of the balloon 12. The necks 20, 22 are fixed to the balloon catheter 12 in fluid tight manner, typically by fusing or bonding with an adhesive or other bonding agent. Suitable methods for fixing the balloon 10 to balloon catheter 12 are well known in the art.

The balloon 10 has an internal chamber 24 which surrounds the portion of the balloon catheter 12 between the necks 20 and 22. The chamber 24 can be filled or emptied, typically with saline solution, via a port 26 in the balloon catheter 12 which communicates with a lumen within the catheter 12, as is known in the art.

The balloon 10 is generally made of a consistent balloon wall material along its entire length, that is its body portion 14, the end cones 16, 18 and the necks 20, 22. Typically, the balloon 10 is formed of a thin and strong polymer material, for example polyamide such as Nylon, polyether block amide such as Pebax(TM), polyethylene terephthalate (PET), polyethylene, polyurethane, among others. It is to be understood that these are only examples of materials suitable for balloon materials and the skilled person will be readily able to identify other appropriate materials. The material of the medical balloon may be compliant, that is of a nature which will stretch upon the application of stress or inflation pressure to the balloon 10, and may also be of a substantially non-compliant material, that is one which is less liable to stretch upon inflation of the balloon 10. It is generally desired to make the wall of the balloon 10 as thin as possible as this increases the wrappability and foldability of the balloon 10 for deployment purposes and in particular in order to reduce the footprint of the deflated balloon catheter for endoluminal delivery through the patient's vasculature and also to be able to treat very small diameter vessels including, for example, the cerebral vessels.

The balloon 10 could be formed of a single layer of material but in other embodiments could be made of a plurality of layers of material, each having different characteristics.

A medical balloon formed with a similar structure as depicted in Figure 1 will generally, when inflated to operating pressures, be smooth on its outer surface and will also be virtually invisible to imaging devices. A medical balloon with a smooth outer wall, as described above, can be quite slippery and as a result can slide within a vessel or across a valve. Moreover, if the medical balloon is used in the deployment of a medical device such as a stent or stent graft, the slippery surface of the balloon can result in the medical device sliding off the balloon during the deployment process, leading to the risk of incomplete deployment of the medical device or deployment of the device in a wrong location in the lumen. Furthermore, a medical balloon having a very simple structure is not suitable for inflation to high operating pressures as such a balloon can be liable to burst, and in particular to burst in a manner which can result in fragmentation of the balloon and potential loss of fragments within the blood stream. Risk of such fragmentation is particularly acute when balloon burst can propagate circumferentially around the balloon wall.

Referring now to Figure 2, this shows a first embodiment of medical balloon 30 mounted on a balloon catheter 32 having, in this embodiment, a generally cylindrical body portion 34, first and second conical end portions 36, 38 terminating in necks 40, 42 respectively. The necks 40, 42 are sealed in fluid tight manner to the catheter 32, in a manner similar to the example of Figure 1. The balloon 30 has an internal chamber 60 equivalent to the chamber 24 of the example of Figure 1.

The medical balloon 30 also includes, in this embodiment, four strips 50 which extend across the entire length of the medical balloon 30, that is along the body portion 34, the end cones 36, 38 and the necks 40, 42. The strips 50, in this embodiment extend parallel to the longitudinal axis of the medical balloon 30, which is typically the longitudinal axis of the catheter 32. The number of strips 50 can vary from the embodiment shown in the Figures. There may, for example be fewer than four or greater than four. It is also not necessary for the strips 50 to extend parallel to the longitudinal axis of the balloon but could extend at angles to this axis, for example helically around the outer perimeter of the balloon. It is, though, preferred that the strips 50 extend substantially parallel to the longitudinal axis of the balloon, for reasons which will become apparent below.

With reference also to Figure 3, each strip 50 overlies the balloon wall 60 and is fixed thereto, for instance by co-extrusion or chemical or heat bonding, so as to create a unitary assembly. Each strip 50 is formed of an amorphous polymer material. The strips are preferably generally flattened and have a height above the perimeter of the balloon wall 60 of between around 0.05 to 0.2 mm. As a result of their reduced height, the strips 50 do not provide any significant scoring or cutting function to the balloon, rather they can in embodiments allow the balloon to retain a generally circular outer perimeter.

In some embodiments, the polymer used in the strips 50 is the same as that used for the wall 60 of the balloon 30, which is the same as that used in the example balloon of Figure 1.

In embodiments, the polymer material used for the strips 50 is harder or more rigid than the material used for the wall of the balloon 60. This could be achieved by selecting a polymer material for the strips 50 which is naturally harder or more rigid than a polymer material used for the balloon wall 60. Amorphous polymer materials have been found to provide rigid elements which can also be easily textured and/or shaped. Some embodiments can therefore use an amorphous polymer material for the strips and a non-amorphous material, preferably including the same polymer, for the balloon wall in order to provide strips of a more rigid material than the balloon wall. One example uses amorphous Nylon 12 such as Grilamid(TM) TR55 as the polymer material for the strips and non-amorphous Nylon 12 as the polymer material for the balloon wall. Other embodiments use different polymers in the amorphous polymer material for the strips and the polymer material for the balloon wall, with the polymer material for the balloon wall less hard or less rigid than the amorphous polymer material of the strips. One example uses amorphous Nylon 6.6 or Nylon 6 as the polymer material for the strips 50 and Nylon 12 as the polymer for the balloon wall 60. Some embodiments use hardening additives in the polymer material of the strips 50 and/or softening additives in the polymer material of the balloon material 60.

It is not excluded that for some embodiments the strips may comprise a polymer material which is softer than the polymer material used for the balloon walls.

Amorphous polymer material has been found not to be as strong as non-amorphous polymer material, so providing the balloon wall with a non-amorphous polymer material means that the strips can provide the desired shape or texture while the balloon wall can provide strength to the balloon.

In some embodiments, the strips 50 have tungsten powder embedded therein, to a ratio of around 80% tungsten to 20% polymer material by weight. Other embodiments may have between 25 to 90% by weight of conductive particulate material, more preferably from 60 to 85% by weight of particulate material.

Below is a reference regarding the effect of density of tungsten on % by volume. Tungsten has a density of 19.35.

| % by Weight | % by Volume |
|---|---|
| 20 | 1.4 |
| 30 | 2.4 |
| 40 | 3.6 |
| 50 | 5.4 |
| 60 | 7.9 |
| 70 | 11.7 |
| 80 | 18.5 |
| 90 | 33.9 |

The strips are configured so that when the balloon is inflated, they have a thickness which is greater than the wall thickness. The thickness of a strip could also be considered to be the height of the strip. Preferably, the thickness of the strips is at least twice, at least four times, or most preferably at least six times the thickness of the wall.

An advantage of the greater thickness of the strips is that they can stop circumferential tears in the balloon wall. As mentioned above, circumferential tears are potentially more problematic than longitudinal tears, and being able to stop circumferential propagation of such tears can minimise the risk of portions of a torn balloon snagging within a vessel or breaking off.

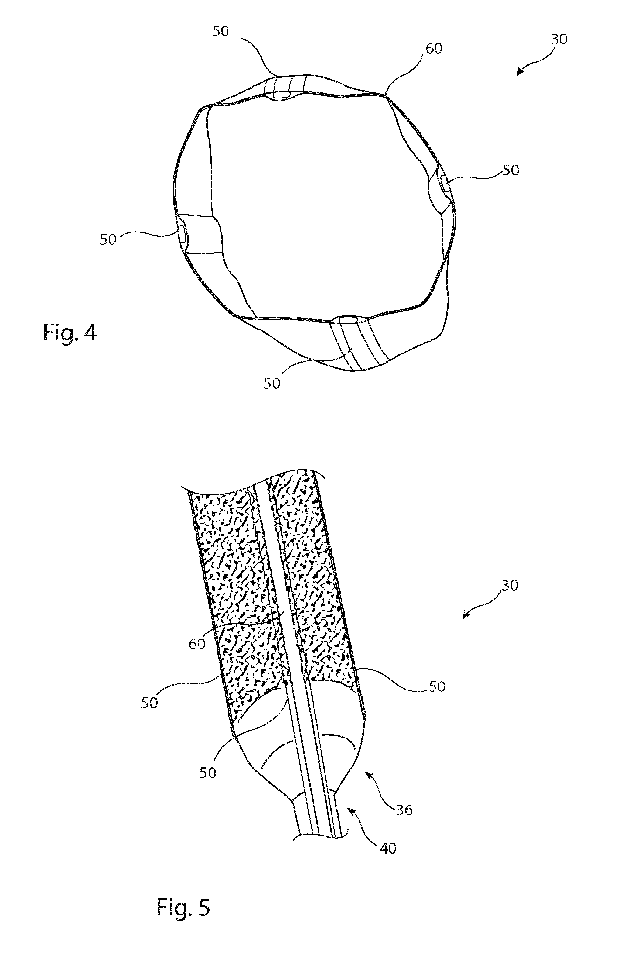

Referring to Figure 4, this shows a diagram of a transverse cross-section of an embodiment of medical balloon 30 equivalent to the sketch in Figure 3. The balloon shown in Figure 4 is in a state in which it can be expected after formation in a mold and before the balloon is inflated to operating pressures. As can be seen, the strips 50 will, as a result of the molding process, generally sit within the outer perimeter of the balloon 30, pushing the balloon wall underlying the strips 50 radially inwardly. Only once the balloon 30 is inflated to operating pressures will the balloon wall adopt a truly annular internal shape, caused by the pressure of the inflation fluid, and will this push the strips 50 to protrude slightly beyond the outer surface of the balloon wall, as described in practice by around 0.05 to around 0.2 millimetres.

In the embodiment of Figures 3 and 4, the outer surface of the strips 50 is textured, in this embodiment being roughened. This is achieved, as explained in detail below, by inflating a raw tubing in a mold having a textured or roughened internal surface.

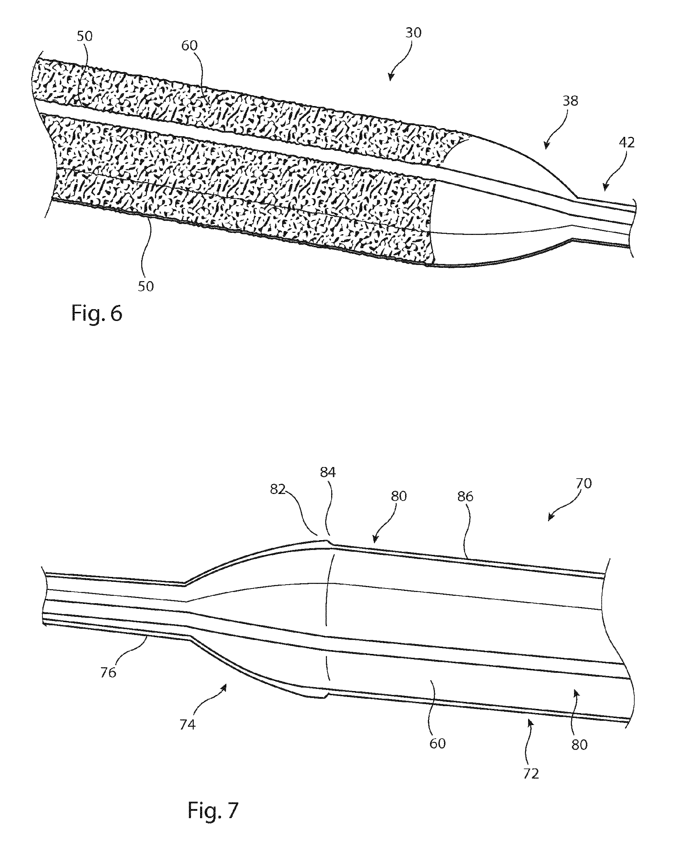

Diagrams of a part of a medical balloon in accordance with the above teachings can be seen in Figures 5 and 6. These diagrams show the balloon 30 in a partially inflated condition and in particular at a pressure below normal operating pressures. In these embodiments, it can be seen that the entirety of the outer surface of the balloon 30 is textured, in this example roughened, including the exposed parts of the balloon wall 60. As can also be seen in the diagrams of Figures 5 and 6, the end cones 16, 18 are not roughened, achieved by having smooth internal surfaces to the manufacturing mold. It is generally not necessary for the conical end portions 36, 38 of the balloon 30 to be textured or roughened as these portions will not generally come into contact with the vessel wall during deployment of the balloon 30. It is not excluded, however, that the conical end portions 36, 38 could be textured or roughened, as could also be the necks 40, 42.

As the balloon wall 60 is relatively thin, preferably as thin as possible in order to optimise the foldability and wrappability of the balloon 30 for delivery purposes, and since the balloon wall 60 in this embodiment is formed of a non-amorphous polymer material and has a generally uniform thickness at least in the body portion 34 of the balloon 30, when the balloon 30 is inflated to a higher pressure, typically to operating pressures, the balloon wall 60 will stretch out any texturing or roughening of its outer surface so as to become flattened. As a result, texturing of the type shown in Figures 5 and 6 will be lost when the balloon is inflated to operating pressures, causing the exposed wall portions 60 to be smooth. This can be seen in the diagram of Figure 7, which is a slightly modified embodiment of the balloon of Figures 5 and 6, as described below. On the other hand, the strips 50 do not lose their texturing or roughening on inflation of the balloon 30 and will therefore retain that texturing during use of the balloon 30. It is believed that this is as a result of the amorphous nature of the material of the strips 50. As a result, embodiments of the medical balloon 30, when inflated to operating pressures, will have a generally smooth outer surface save for the texturing of the strips 50. This enables the strips 50 to provide surface texture to the balloon 30 to stop slippage in use, assisting in holding the balloon 30 in position within the vessel wall or across a valve and/or in holding a medical device securely on the balloon 30 during deployment of the device.

The structure shown in Figures 2 to 7 has an additional advantage in that the strips 50 can exhibit a greater tear resistance compared to the exposed wall of the balloon, with the result that any tear of the balloon 30 will generally not propagate circumferentially through the strips 50. Any tear propagation will as a result tend to move in a longitudinal direction of the balloon 30, which carries a much lower risk of fragmentation of the balloon and loss of balloon fragments into the blood stream. As a result, it is possible to use a balloon wall 60 of a thickness optimal for the intended pressure without having to use the balloon wall of greater thickness than is actually required.

It will be appreciated in particular having regard to Figures 2 to 6 that the balloon 30 retains a high degree of foldability and wrappability. Any increase in volume of material as a result of the strips 50 is minimal and does not impinge upon that foldability or wrappability.

Referring again to Figure 7, there is an embodiment of medical balloon 70 which has very similar characteristics to the embodiment of Figures 2 to 6, differing solely in the structure of the strips 80. More specifically, the balloon 70 includes a balloon body portion 72, end cones 74 and necks 76, similar to example in Figure 1 and embodiments of Figures 2 to 6. The medical balloon 70 also includes a plurality of strips 80, made of the same material as the strips 50 of the previous embodiments. In this particular embodiment, though, the strips 80 are not necessarily textured in the manner of the strips 50 shown in Figures 3, 5 and 6 (although they may be so textured). In this particular embodiment, the strips 80 include a raised portion 82 extending up to the end of the end cone 74 and providing a shoulder 84 adjacent a portion 86 of the strip 80 extending across the body portion 72. The strip portion 86 has a lower profile than that of the portion 82 and shoulder 84. The other end of the balloon 70 will have a shape consistent with the end visible in Figure 7.

The strips 80 can be produced by reducing their height, for example by flattening, in the zones in which they are to be recessed and leaving the other zones untouched during the blowing process, such that they remain raised.

This shape of the strips 80, and in particular the zone delimited by the shoulders 84, provides a holding zone for holding a medical device, for example a stent or stent graft, over the body portion 72 of the medical balloon 70 and for minimising any risk of the medical device sliding off the balloon 70. The medical device can therefore be held securely to the medical balloon until fully deployed against the internal surface of a vessel wall and the balloon deflated.

The embodiment of Figure 7 has a single recessed region 86 along the length of each strip 80, although in other embodiments there may be a plurality of recessed regions along each strip 80. Disposed between each pair of adjacent recessed regions 86 there would typically be provided a non-compressed strip portion to provide shoulders similar to the shoulders 84 shown in Figure 7. A plurality of recessed regions, aligned among the adjacent stripes 80, can be useful in holding a segmented device or a plurality of separate medical devices securely on the delivery balloon.

The strips 80 preferably also have all of the advantages of the embodiments described above.

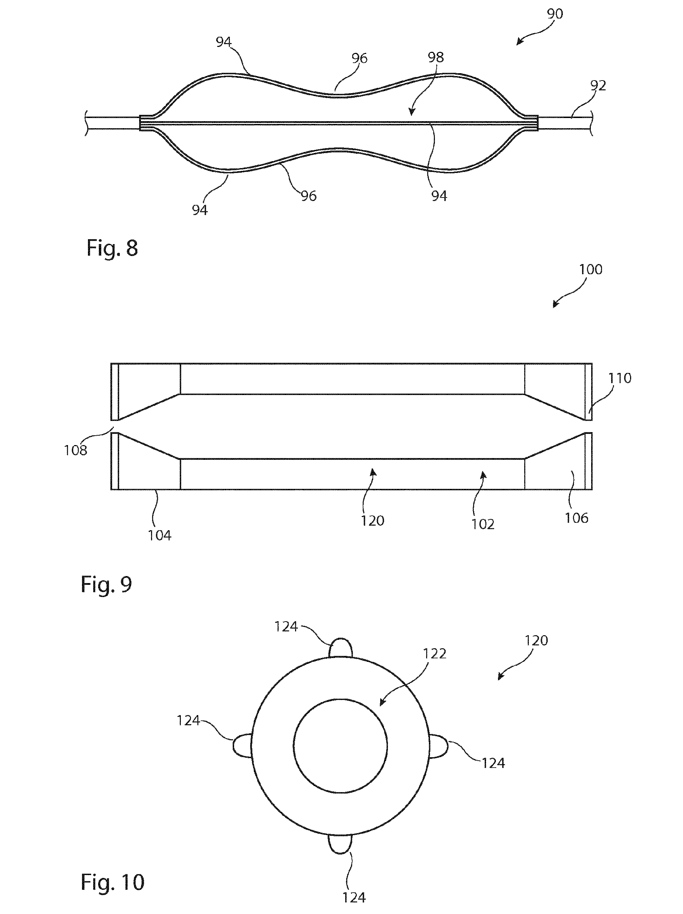

Referring now to Figure 8, this shows another embodiment of medical balloon 90 similar to the embodiments described above and fitted to a balloon catheter 92. The balloon 90 has a plurality of strips 94 along extending its length and generally parallel to the longitudinal axis of the balloon 90. In this example the strips 94 are shaped to have a waist 96 around the centre portion of the balloon 90. A waist 96 of this nature, and indeed any other shape to the strips 94, can be achieved by use of a mold having an internal surface to that shape. It has been found that the strips 94 will retain their contoured shape even on inflation of the balloon 90 to full operating pressures. This is achieved as a result of the relative stiffness of the strips 94 compared to the flexibility of the exposed portions 98 of the balloon wall, which will stretch preferentially to the strips 94. A balloon of this nature can be particularly useful for valve treatment, for deployment of medical devices and so on.

It will be appreciated, that the strips 94 will have a structure and characteristics very similar to those of the previously described embodiments.

The skilled person will appreciate also that by having a consistent balloon wall underlying the strips 50, 80, 94 ensures that the balloon wall retains its structural integrity by being complete and uninterrupted. The strips do not cause any loss of that integrity. On the other hand, structures in which the balloon wall has embedded therein particulate material can be weakened by that material and therefore are more prone to bursting at high pressures.

Referring now to Figure 9, this shows in schematic form an example of mold 100 for producing a medical balloon as taught herein. The mold includes a mold chamber formed of a main body portion 102 and end portions 104, 106 which, in this example are conical in shape, and end necks 108, 110. The mold 100 is typically constructed as a plurality of components which can be separated in order to remove a formed balloon from inside.

The mold 100 has an internal surface 120 which has a shape and texturing consistent with intended shape and texturing of the balloon.

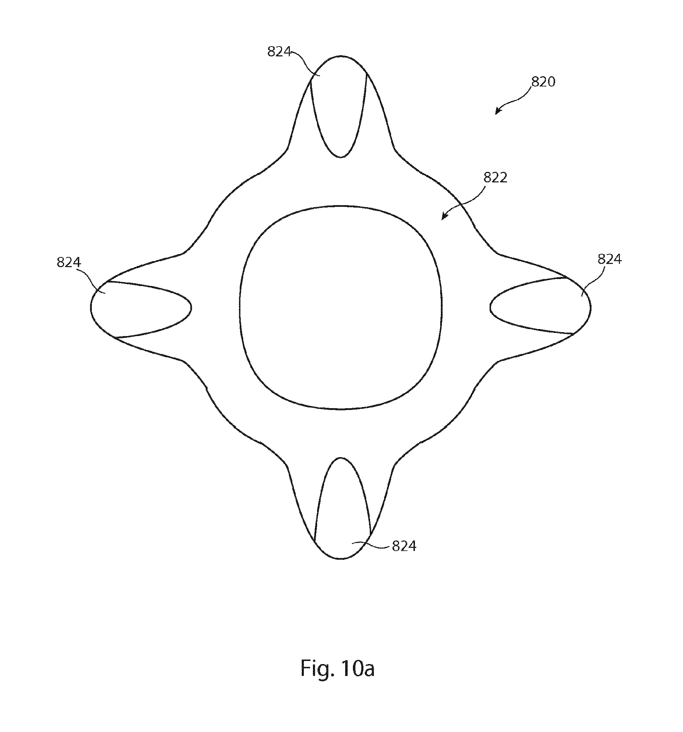

Figures 10 to 12 show examples of different structures of raw tubing for producing balloons having strips with different characteristics. While the schematic drawings show the strips being made entirely of amorphous polymer material, it is to be understood that in practice co-extrusion of the ribs and tubing for forming the raw balloon form can cause the polymer material of the raw tubing and of the ribs to intermingle, for example for some of the polymer of the raw tubing to flow into the volume of the ribs during the extrusion process. This does not affect the function or structure of the raw balloon form or of the finished balloon.

Figure 10 shows in schematic form an example of raw tubing 120 for the production of a medical balloon as taught herein. It will be appreciated that what is shown in Figure 10 is a cross-sectional view of such a raw tubing. This includes a tube 122 of balloon wall material and a plurality of ribs 124 of the material which will form the strips taught herein. The raw tubing could be formed as a co-extrusion of the tube 122 and ribs 124. For example, the die in the extruder can have grooves and can put the ribs on the tube thereby extruding the raw tubing in a single process.

The raw tubing 120 is in practice fed into the mold 100 so that it extends through the necks 108, 110 and fixed into the mold 100. One end of the raw tubing 120 is closed, while inflation fluid is pumped into the other end of the raw tubing 120. The mold 100 is heated to a temperature sufficient to cause softening of the tube material 122 which, under the inflation pressure will then expand up to the internal surfaces 120 of the mold 100. During this expansion process, the ribs 124, which will also be softened by the applied heat, will be pressed against the internal walls of the mold 100, thereby causing the ribs 124 to flatten as shown in Figure 4. Moreover, any texturing or shaping of the internal surface 120 of the mold 100 will be applied to the outer surface of the balloon 120 and in particular to the ribs 124 forming the strips of the balloon.

Where the balloon and in particular the strips are to have a non-uniform shape, for example as that shown in Figure 8, the mold chamber will have an internal wall shaped to that desired shape. The raw tubing and strips will therefore be blown to that shape of the mold.

Figure 10A shows in schematic form another example of raw tubing 820 shown in cross-sectional view. This includes a tube 822 of balloon wall material and a plurality of ribs 824 of the material which will form the strips taught herein. The raw tubing could be formed as a co-extrusion of the tube 822 and ribs 824. A balloon can be produced from the raw tubing 820 as described in respect of Figure 10.

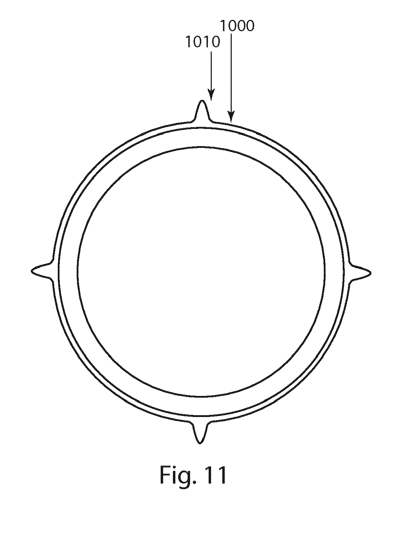

In some embodiments only the strips include the amorphous polymer material used for the strips. However, in other embodiments, such as shown in Figure 11, the raw tubing for the balloon can include a layer 1000 disposed on the outer surface of the tube. The layer 1000 is made of an amorphous polymer material as described above. The strips 1010 are formed as part of the layer. The layer 1000 is substantially smooth circumferentially around the tubing portion with the exception of the strips 1010 which extend radially outwardly and thereby provide discontinuities in the outer surface of the layer 1000. It will be appreciated that the cross-section shown in Figure 11 is before the strips have been flattened by the mold.

Providing the strips as part of the layer 1000 has advantages in that it is easier to co-extrude the balloon in this form. However, because the layer 1000 includes amorphous polymer material, it can be weaker than the tubing portion, which is preferably made of non-amorphous material, so the sections of the layer 1000 between the strips can adversely affect the strength of the balloon. In other words, in embodiments where only the strips include the amorphous polymer material, the balloon can be stronger.

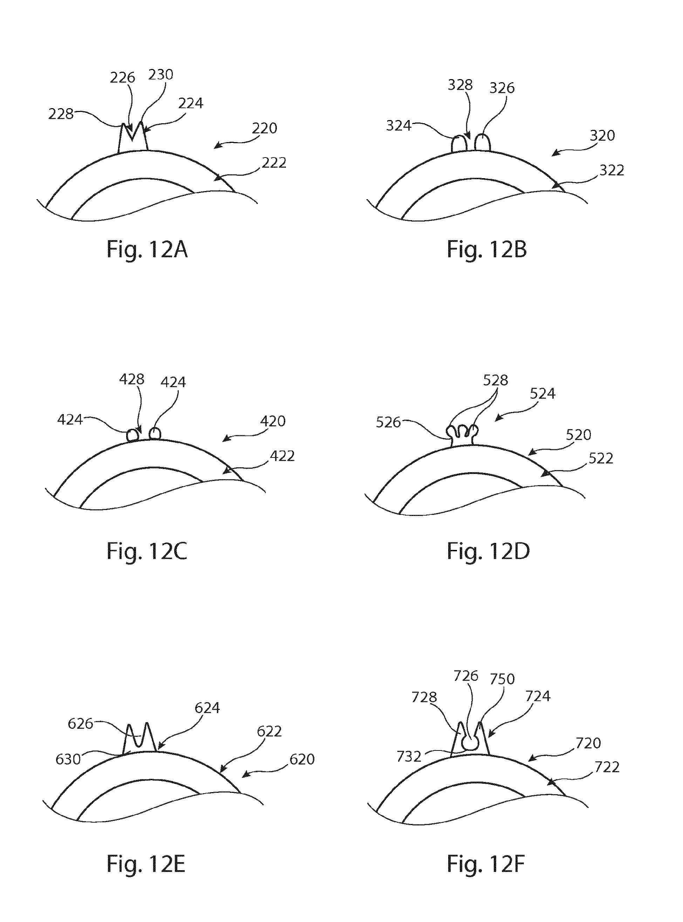

Referring now to Figures 12A-12F, these show schematically different forms of raw tubing for producing strengthened medical balloons having particular strengthening and flexibility characteristics.

With reference first to Figure 12A, this shows in schematic form a raw tubing 220 having a single or multi-layered balloon wall 222 to which there is attached, bonded or co-extruded, a plurality of ribs 224, which a single one is visible in Figure 12A. The ribs 224 extend in the longitudinal direction of the raw tubing 220. In contrast with the example of Figure 10, the rib 224 has a longitudinally extending V-shaped groove 226, which extends for the entire length of the rib 224. In this embodiment, it is preferred that during the process of blowing a balloon from the raw tubing 220, the ribs 224 are kept intact for at least a part of their length. The groove 226, which is preferably of a significant depth, at least 50% of the height of the ribs 224, will cause the ribs 224 to be flexible in a transverse direction, specifically such that the apices 228, 230 can move closer to one another during deflation and folding of the balloon. This facilitates the folding and wrapping of the balloon for deployment purposes.

Referring now to Figure 12B, this shows another embodiment of raw tubing 320 having a single or multi-layer tubing 322 of the types previously disclosed. In place of a single rib in each circumferential location of the raw tubing 320 in which it is desired to have strengthening elements, the embodiment of Figure 12B has a pair of smaller ribs 324, 326 which are disposed adjacent one another with a small gap 328 therebetween. The ribs 324 can be at least partially flattened during the blowing of the raw tubing 320 to form a medical balloon, but will retain a gap 328 therebetween. The ribs 324, 326 can function together in place of a single larger rib 124, 224, as per the embodiments of Figures 10 and 12A. However, the gap 328 between the neighbouring ribs 324, 326 provide enhanced folding characteristics to the balloon.

The embodiment of Figure 12C has a pair of small ribs 424 of wire like form attached to the wall 422 of the raw tubing 420, in manner similar to the example of Figure 12D. The pair of wire-like lines 424 will act in place of each of the strips of the previously described embodiments of Figures 1 to 8, 9 and 12A.

In Figure 12D there is shown an embodiment of raw tubing 520 having a tube wall 522 as previously described and a plurality of rib elements 524 extending longitudinally along the outer surface of the tubing 522. Each rib 524 has a base 526 and a plurality of raised protrusions 528 spaced from one another.

Referring now to Figure 12E, this shows another embodiment of raw tubing 620 to which there is attached to the outer surface of the tube wall 622 a plurality of ribs 624, of which only one is again visible in the drawing. Each rib 624 has a base 630 attached to the tube wall 622 and a groove 626 extending to close to the bottom surface 630 of the rib 624. This groove 626, which runs along the longitudinal length of the rib 620, will enhance the flexibility of the balloon and in particular allow the balloon to fold around the ribs 624.

Yet another version is shown in Figure 12F, in which the raw tubing 720 is provided with a rib 724 having a recess 726 therein which has V-shaped walls 728, 730 which then open out to a circular cylindrical channel 732.

It will be appreciated that in the various embodiments of Figures 12A-12F the balloon wall can be made of any of the materials and any of the structures disclosed herein, and the ribs can be made of any of the materials disclosed herein.

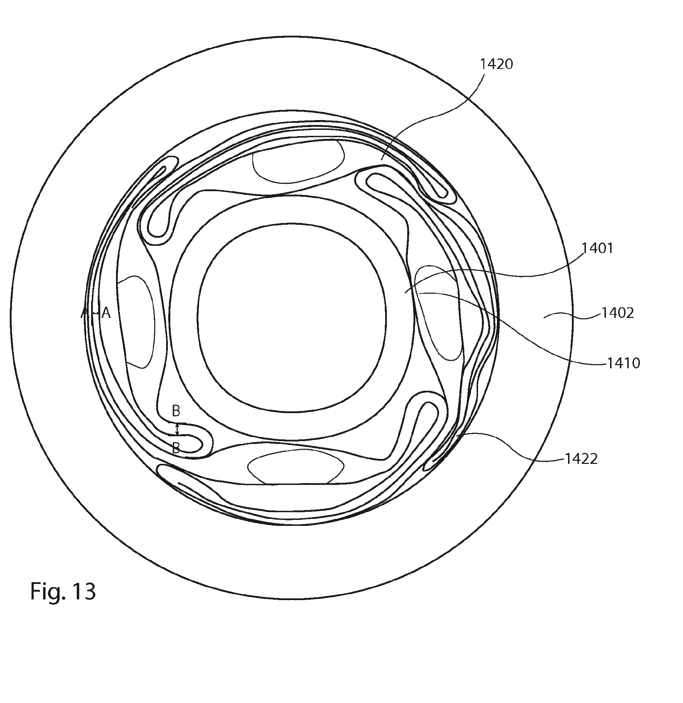

Figure 13 shows a cross section of a balloon 1420 similar to the embodiment of Figure 4 folded inside a delivery device. The delivery device includes a first catheter 1401 and a coaxial second catheter or sheath. The balloon is folded within an annular space between the two catheters. In this embodiment, the inner diameter of the second catheter or sheath 1402 is 2.124mm (0.084in). Figure 13 also shows the greater thickness of the strips 1410 as compared to the balloon wall 1422. As can be seen in Figure 13, the thickness of the balloon wall varies in this embodiment. For example, the distance A-A is 0.031 mm and the distance B-B is 0.053mm.

It will be appreciated that the balloon per se may be made of a single layer of polymer material but in other embodiments may be a multi-layer structure. Different layers can be made of similar or complementary materials, in a manner which will be apparent to the person skilled in the art.

For embodiments that include a heat conductive powder, tungsten is a preferred material for the powder since it is radiopaque, although any other heat conducted particulate material can be used. Other examples include gold, plasmin, silver and palladium. Of course, it is not necessary to use a single material for the particulates.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosure in the abstract accompanying this application is incorporated herein by reference.

The disclosures in GB 1415424.9 and GB 1419873.3, from which this application claims priority, are incorporated herein by reference.

## Claims

1. A medical balloon, including:
a balloon body member, the balloon body member having first and second ends, a longitudinal dimension and a body length, and a balloon wall formed of polymer material;
at least one strip extending between the first and second ends of the body member, the at least one strip comprising amorphous polymer material; the at least one strip being at least one of textured and shaped.

2. A medical balloon according to claim 1, wherein the at least one strip is fixed to and disposed in overlying relationship on the balloon wall.

3. A medical balloon according to claim 1 or 2, wherein the balloon wall comprises non-amorphous polymer material.

4. A medical balloon according to any preceding claim, including a plurality of said strips.

5. A medical balloon according to any preceding claim, wherein the or each strip extends parallel to the longitudinal direction of the balloon body member.

6. A medical balloon according to any preceding claim wherein the or each strip is shaped to include at least one radial departure from a longitudinal straight line between the first and second ends of the balloon body member.

7. A medical balloon according to any preceding claim, wherein the or each strip has a flattened outer surface.

8. A medical balloon according to any preceding claim, wherein the or each strip comprises a polymer material having a greater hardness than the hardness of the polymer material forming the balloon body member.

9. A medical balloon according to any one of claims 1 to 7, wherein the or each strip has an equal or greater softness than a softness of the balloon body member.

10. A medical balloon according to any preceding claim, wherein the or each strip comprises Nylon and/or wherein the balloon body member comprises Nylon.

11. A medical balloon according to any preceding claim, wherein the or each strip has a concave shape between the first and second ends of the balloon.

12. A medical balloon according to any preceding claim, wherein the or each strip has a greater tear resistance than a tear resistance of the balloon wall.

13. A method of forming a medical balloon including the steps of:
forming a raw balloon tubing by:
a) forming a tube of balloon material;
b) applying to the tube of balloon material at least one strip comprising amorphous polymer material;
placing the raw balloon tubing in a mold having an inner surface in the shape of a medical balloon;
heating and inflating the raw tubing in the mold, thereby to cause the raw tubing to inflate to the inner surface of the mold, wherein the at least one strip is flattened between the mold surface and the balloon material, as well as being at least one of textured and shaped; and
removing the formed balloon from the mold.

14. A method according to claim 13, wherein the tube and at least one strip are coextruded and/or wherein the inner mold surface is roughened or textured, whereby inflation of the raw tubing causes the outer surface of the balloon and of the or each strip to be roughened or textured.

15. A method according to claim 13 or 14, wherein the inner mold surface has a varying diameter along a length thereof, wherein the raw tubing inflates to a varying diameter so as to cause the or at least one of the strips to have a non-straight inflated shape.
